# EUROPEAN PATENT APPLICATION

(11) **EP 4 295 817 A1**
(43) Date of publication of application: **27.12.2023**
(21) Application number: 22756252.7
(22) Date of filing: 17.02.2022
(51) Int. Cl.: A61F 2/915, A61F 2/958

(54) **STENT AND STENT DELIVERY SYSTEM**

(30) Priority: 19.02.2021 JP 2021024885
(71) Applicant: TERUMO Kabushiki Kaisha, Tokyo 151-0072 (JP)
(72) Inventor: KAITA, Shohei, Fujinomiya-shi, Shizuoka 418-0015 (JP); NAKAYA, Shinsuke, Fujinomiya-shi, Shizuoka 418-0015 (JP)
(74) Representative: Casalonga
(86) International application number: PCT/JP2022/006311
(87) International publication number: WO 2022/176934

(57) **Abstract**

To provide a stable stent-retaining force with respect to a balloon and make a local radial force uniform even at the time of expansion to a larger diameter.

In a stent 100, a second distance L2, which is a separation distance in the circumferential direction between an extreme end portion in the long-axis direction of a second bent portion 35 connected to an end portion on one end side of a second linear portion 34 and an extreme end portion in the long-axis direction of the second bent portion 35 connected to a portion on the other end side of the second linear portion 34, is longer than a first distance L1 which is a separation distance in the circumferential direction between an extreme end portion in the long-axis direction of a first bent portion 32 connected to an end portion on one end side of any of first linear portions 31 and an extreme end portion in the long-axis direction of the first bent portion 32 or the second bent portion 35 connected to an end portion on the other end side of the first linear portion 31, and the number of the first linear portions 31 disposed in a wavy unit 33 is four or more.

## Description

### Technical Field

The present invention relates to a stent and a stent delivery system.

### Background Art

A stent is a medical tool that is sent to a lesion in a lumen of a living body by a stent delivery system and then placed to expand the lesion such as a stenosed site or an occluded site and secure a passage thereof in order to treat various diseases caused by stenosis or occlusion of the lumen such as a blood vessel. The stent has a gap formed by a strut, which is a linear component, and is shaped to have a cylindrical outer periphery that can be radially enlarged and reduced in diameter.

Stents are divided into a balloon-expandable type and a self-expandable type depending on a function and a placement method. Among them, a stent of the balloon-expandable type is attached to a balloon catheter in a crimped (diameter-reduced) state, sent (delivered) to a target lesion, and then placed in a lumen of a living body. The stent needs to be prevented from being detached from a balloon during the delivery. Therefore, the stent is subjected to crimping (pressure crimping) while inflating the balloon at a low pressure such that the balloon protrudes outward from an inner surface of the stent from a gap between struts when being crimped. Prior to the pressure crimping, the balloon is subjected to pre-inflation to wrinkle an outer surface of the balloon such that the balloon easily protrudes from the gap of the strut.

However, wrinkles generated at the time of pre-inflation of the balloon have random shapes, and thus, it is difficult to cause the balloon to protrude from a target position between the struts at the time of the pressure crimping. Therefore, when the balloon catheter in which the stent is crimped is mass-produced, there is a large individual difference in a stent-retaining force between the balloon catheters.

In order to solve the above problem regarding the stent-retaining force, there is known a stent disclosed in Patent Literature 1 below. The stent in Patent Literature 1 below is formed such that intervals in the
circumferential direction of a strut forming a wavy annular member are not uniform, and an interval between an inclined curved line portion and a first inclined straight-line portion and an interval between the inclined curved line portion and a second inclined straight-line portion are larger than an interval between a parallel straight-line portion and the first inclined straight-line portion and an interval between the parallel straight-line portion and the second inclined straight-line portion. Since the intervals in the circumferential direction of the strut are not uniform in the stent of Patent Literature 1, a balloon is less likely to protrude from a region where the interval in the circumferential direction of the strut is small, but the balloon is likely to protrude from a region where the interval in the circumferential direction of the strut is large, as compared with a case where intervals in the circumferential direction of strut are uniform. Therefore, variations in whether the balloon protrudes or not at the time of pressure crimping is reduced in the stent of Patent Literature 1, and a stable stent-retaining force can be exhibited.

### Citation List

### Patent Literature

Patent Literature 1: JP 2008-86463 A

### Summary of Invention

### Technical Problem

As a stent expands to a larger diameter, an interval between adjacent struts in the circumferential direction increases. At this time, in an annular member formed of the strut, an amplitude, which is an axial distance, decreases, and a shape changes from a wavy shape to a straight shape. Therefore, a distance from any position where there is no strut to the strut is generally closer in a case where the strut has a wavy shape than that in the case where the strut has a straight shape.

For this reason, local distribution of a radial force (radial compressive force resistance) acting on an intravascular surface can be more uniform in a case where the annular member has a wavy shape than that in a case where the annular member has a straight shape. Furthermore, when a stent is of a drug-eluting type, more uniform elution is possible in a case where an annular member has a wave shape than in a case where the annular member has a straight shape in terms of local distribution of a drug. As the local distribution of the radial force or the drug becomes more uniform, clinical risk such as restenosis of a lesion can be reduced more.

As described above, it can be said that it is preferable to keep a shape of a strut in a wavy shape in consideration of the clinical risk even when a stent in a larger diameter is applied. Therefore, there is a demand for a stent capable of reducing the clinical risk by making a local radial force uniform even when being applied in a large diameter while having an excellent stent-retaining force of the stent of Patent Literature 1.

At least one embodiment of the present invention has been made in view of the above circumstances, and specifically, provides a stent, capable of achieving a stable stent-retaining force with respect to a balloon and making a local radial force uniform even at the time of expanding to a larger diameter, and a stent delivery system.

### Solution to Problem

A stent according to the present embodiment is a stent that is expandable and contractible in a radial direction of a cylindrical shape, the stent including: a plurality of annular members formed in an annular shape by a linear component folded in a wave shape and disposed along a long-axis direction of the cylindrical shape to form the cylindrical shape; and a link portion connecting the annular members adjacent to each other. The annular member is formed by disposing a plurality of basic units in a circumferential direction of the cylindrical shape and connecting the basic units adjacent to each other by a second bent portion. The basic unit includes: a wavy unit which has a plurality of first linear portions extending from a proximal end side to a distal end side in the long-axis direction and disposed continuously in the circumferential direction and a first bent portion connecting end portions on the proximal end side or the distal end side of two of the first linear portions adjacent in the circumferential direction; a second linear portion extending from the proximal end side to the distal end side in the long-axis direction and disposed at a position adjacent to the first linear portion in the circumferential direction; and the second bent portion connecting end portions on the proximal end side or the distal end side of the first linear portion and the second linear portion adjacent to each other in the circumferential direction. A second distance, which is a separation distance in the circumferential direction between an extreme end portion in the long-axis direction of the second bent portion connected to an end portion on one end side of the second linear portion and an extreme end portion in the long-axis direction of the second bent portion connected to an end portion on another end side of the second linear portion, is longer than a first distance which is a separation distance in the circumferential direction between an extreme end portion in the long-axis direction of the first bent portion connected to an end portion on one end side of any of the first linear portions and an extreme end portion in the long-axis direction of the first bent portion or the second bent portion connected to an end portion on another end side of the first linear portion. The number of the first linear portions disposed in the wavy unit is four or more.

A stent delivery system according to the present embodiment is a stent delivery system including a stent having the above-described configuration and a balloon catheter having an inflatable and deflatable balloon, and the stent is retained in close contact with the deflated balloon in a contracted state, and the balloon protrudes outward from a radial position of an inner surface of the stent only at a position between the first linear portion and the second linear portion.

### Advantageous Effects of Invention

According to one embodiment of the present invention, it is possible to achieve the stable stent-retaining force with respect to the balloon and to make the local radial force uniform at the time of expansion to a larger diameter.

### Brief Description of Drawings

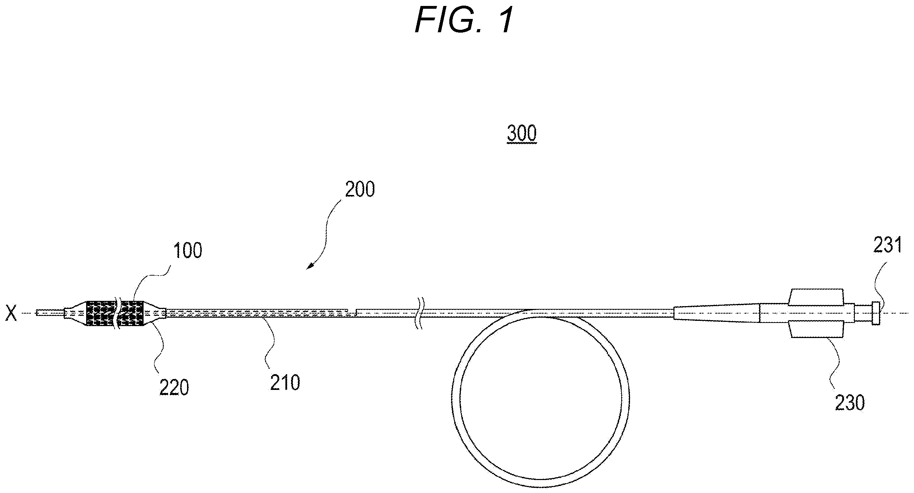

Fig. 1 is a schematic plan view of a stent delivery system according to the present embodiment.
Fig. 2 is a developed view in which a part of an outer periphery of a stent according to the present embodiment is linearly cut along a long-axis direction and developed.
Fig. 3 is a view illustrating a state of a basic unit forming an annular member of the stent according to the present embodiment before stent expansion.
Fig. 4 is a view illustrating a state of the basic unit of the stent according to the present embodiment after the stent expansion.
Fig. 5A is a partially enlarged view of a first bent portion, a link portion, and the vicinity thereof of the stent according to the present embodiment.
Fig. 5B is a partially enlarged view of a second bent portion and the vicinity thereof of the stent according to the present embodiment.
Fig. 6 is a view illustrating a state of the link portion and the peripheral portion thereof of the stent according to the present embodiment before the stent expansion.
Fig. 7 is a view illustrating a state of the link portion and the peripheral portion thereof of the stent according to the present embodiment after the stent expansion.

### Description of Embodiments

Hereinafter, a mode for carrying out the present invention will be described in detail with reference to the drawings. An embodiment herein is illustrated to embody the technical idea of the invention and does not limit the invention. Furthermore, other modes, examples, operation techniques, and the like, that can be implemented by those skilled in the art without departing from the gist of the present invention are all included in the scope and gist of the present invention and included in the invention described in the claims and the scope of equivalents thereof.

Moreover, for convenience of illustration and ease of understanding, the drawings attached to the present specification may be schematically represented by changing a scale, an aspect ratio, a shape, and the like from actual ones as appropriate, but are merely examples, and do not limit the interpretation of the present invention.

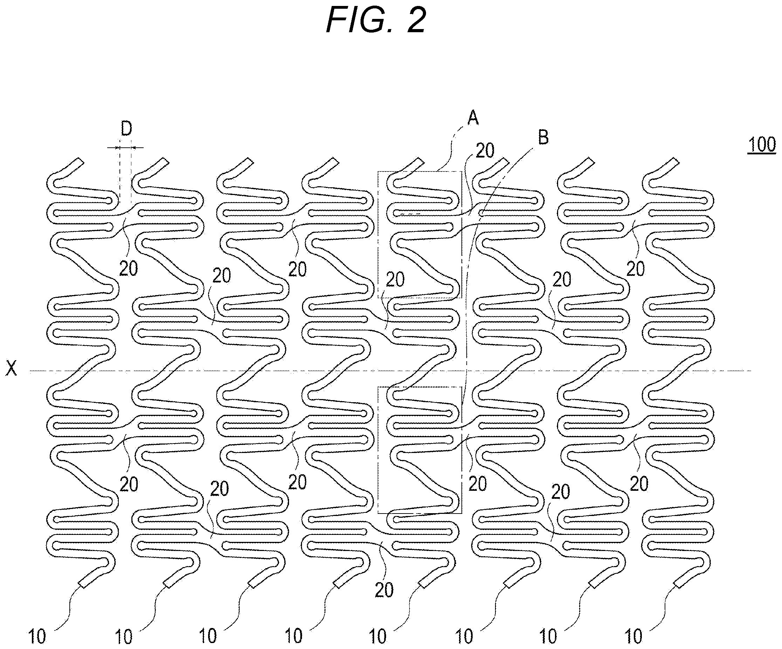

In the present specification, a long-axis direction of a stent 100 (a longitudinal direction of the stent 100, for example, the left-right direction in the drawing along a central axis X (two-dot chain line) of the stent 100 illustrated in Fig. 2) having a cylindrical shape and formed of a strut, which is a linear component, is simply referred to as the "long-axis direction". Furthermore, a circumferential direction (the vertical direction in the drawing) of the stent 100 (an annular member 10) illustrated in Fig. 2 is simply referred to as the "circumferential direction", and a radial direction of the stent 100 (the annular member 10) is simply referred to as the "radial direction". Furthermore, a side to be inserted into a living body is referred to as a "distal end side", and a side which is opposite to the distal end side and on which a surgeon operates a medical device is referred to as a "proximal end side".

Note that, in the following description, ordinal numerals such as "first" and "second" will be given, but are used for convenience and do not define any order unless otherwise specified.

The stent 100 according to the present embodiment is used to treat a stenosed site or an occluded site generated in a blood vessel, a bile duct, a trachea, an esophagus, a urethra, or other lumens of a living body. The stent 100 is a so-called balloon-expandable stent that is mounted (attached) in a state of being crimped to a balloon 220, sent to a lesion, and then expanded and placed at the lesion.

A stent delivery system 300 includes the stent 100 and a balloon catheter 200. The balloon catheter 200 is used to send the stent 100 in a contracted state to the lesion, expand the stent, and place the stent at the lesion.

The balloon catheter 200 includes an elongated catheter body 210, the balloon 220 provided at a distal end of the catheter body 210, and a hub 230 fixed to a proximal end of the catheter body 210.

The catheter body 210 includes an outer tube and an inner tube disposed inside the outer tube.

An expansion lumen through which an expansion fluid for expanding the balloon 220 flows is formed inside the outer tube. A distal end portion of the outer tube is fixed to a proximal end portion of the balloon 220. A proximal end portion of the outer tube is fixed to the hub 230.

A guide wire lumen into which a guide wire is inserted is formed inside the inner tube. A distal end portion of the inner tube penetrates the inside of the balloon 220 and is opened on the distal end side of the balloon 220. A proximal end portion of the inner tube penetrates a side wall of the outer tube on the proximal end side of the balloon 220 and is fixed to the outer tube.

A constituent material of the catheter body 210 is preferably a material having a certain degree of flexibility, and examples thereof include polyolefins such as polyethylene, polypropylene, polybutene, an ethylenepropylene copolymer, an ethylene-vinyl acetate copolymer, and an ionomer, or a mixture of two or more kinds thereof, thermoplastic resins such as a polyvinyl chloride resin, polyamide, a polyamide elastomer, polyester, a polyester elastomer, polyurethane, and a fluororesin, a silicone rubber, a latex rubber, and the like.

The balloon 220 is a member that pushes and expands a stenosed site by expanding inside the stenosed part, for example. The distal end side of the balloon 220 is fixed to an outer wall surface of the inner tube. The proximal end side of the balloon 220 is fixed to an outer wall surface of the distal end portion of the outer tube. Therefore, the inside of the balloon 220 communicates with the expansion lumen formed in the outer tube. The balloon 220 allows inflow of the expansion fluid from a proximal opening 231 through the expansion lumen. The balloon 220 is expanded due to the inflow of the expansion fluid, and is contracted and folded by discharging the expansion fluid that has flowed therein.

A constituent material of the balloon 220 is preferably a flexible material that expands and contracts when the expansion fluid flows in and out, and examples thereof include polymer materials such as a polyolefin, a crosslinked polyolefin, polyester, a polyester elastomer, polyvinyl chloride, polyurethane, a polyurethane elastomer, polyphenylene sulfide, polyamide, a polyamide elastomer, and a fluororesin, a silicone rubber, a latex rubber, and the like. The constituent material of the balloon 220 is not limited to a mode in which the above-described polymer material is used alone, and a film in which the above-described polymer materials are appropriately laminated may be applied. Furthermore, the expansion fluid may be a gas or a liquid, and examples thereof include gases such as a helium gas, a CO₂ gas and an O₂ gas, and liquids such as physiological saline and an X-ray contrast agent.

The hub 230 includes the proximal opening 231 communicating with the expansion lumen of the outer tube. The proximal opening 231 functions as a port through which the expansion fluid flows in and out.

A constituent material of the hub 230 is not particularly limited, and examples thereof include thermoplastic resins such as polyethylene, polyurethane, polyester, polypropylene, polycarbonate, polyamide, polysulfone, polyarylate, and a methacrylate-butylene-styrene copolymer.

Next, the stent 100 will be described with appropriate reference to Figs. 2 to 7. In Figs. 2 to 7, the right side in the drawing is referred to as the "proximal end side", and the left side in the drawing is referred to as the "distal end side".

The stent 100 is formed of a strut, which is a linear component, and includes a plurality of annular members 10 arranged in the long-axis direction and link portions 20 each connecting the annular members 10 adjacent to each other in the long-axis direction as illustrated in Fig. 2. The stent 100 is formed in a cylindrical shape in which the annular members 10 adjacent to each other are connected by the link portion 20. Note that any component of the stent 100 is referred to as the strut in the present specification.

As illustrated in Fig. 2, the annular member 10 is formed by forming the wavy strut, repeatedly folded in a zigzag manner along the circumferential direction, into an annular shape. Phases of the plurality of annular members 10 in the circumferential direction are aligned.

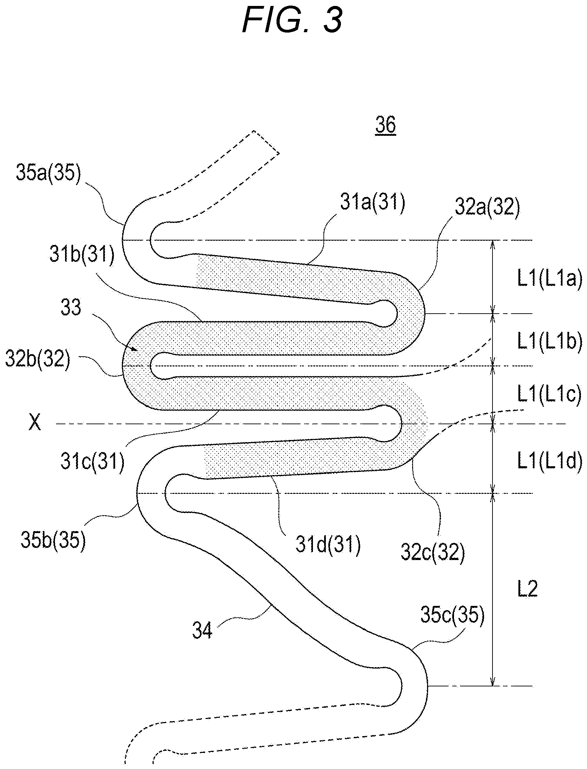

As indicated by the dot-hatched area in Fig. 3, a wavy unit 33 includes: a plurality of first linear portions 31 (31a to 31d) that extend from the proximal end side to the distal end side in the long-axis direction and are disposed continuously in the circumferential direction; and first bent portions 32 (32a to 32c) that connect end portions on the proximal end side or the distal end side of two first linear portions 31 adjacent in the circumferential direction. The first linear portion 31 and the first bent portion 32 are connected to be continuous in a wave shape along the circumferential direction.

The first linear portion 31 has a substantially straight shape, and four or more first linear portions are disposed continuously along the circumferential direction with distal ends facing the distal end side or the proximal end side in the long-axis direction. In the present embodiment, four first linear portions 31 are continuously disposed along the circumferential direction as illustrated in Fig. 3.

Among the first linear portions 31, the first linear portion 31a and the first linear portion 31d adjacent to second linear portions 34, respectively, are disposed in postures inclined at predetermined angles with respect to the long-axis direction. The first linear portion 31b and the first linear portion 31c disposed between the first linear portion 31a and the first linear portion 31d are disposed substantially parallel to the long-axis direction. The first linear portion 31c functions as an "axially parallel linear portion" that is parallel to the long-axis direction before and after expansion of the stent 100.

The first bent portion 32 connects the end portions on the proximal end side or the distal end side of the two first linear portions 31 adjacent in the circumferential direction to form the wavy unit 33. In the present embodiment, three first bent portions 32 are disposed in the wavy unit 33 along the circumferential direction as illustrated in Fig. 3. As illustrated in Fig. 3, the first bent portion 32 includes the first bent portion 32a connecting the first linear portion 31a and the first linear portion 31b, the first bent portion 32b connecting the first linear portion 31b and the first linear portion 31c, and the first bent portion 32c connecting the first linear portion 31c and the first linear portion 31d.

The basic unit 36 includes: the wavy unit 33 extending to the proximal end side or the distal end side in the long-axis direction; the second linear portion 34 disposed at a position adjacent to the wavy unit 33 in the circumferential direction; and second bent portions 35 each connecting end portions on the proximal end side or the distal end side of the wavy unit 33 and the second linear portion 34 adjacent in the circumferential direction.

The second linear portion 34 has an S-shaped curved shape and is disposed adjacent to the wavy unit 33 in the circumferential direction. The distal end side or the proximal end side of the second linear portion 34 is connected, via the second bent portion 35, to the distal end side or the proximal end side of the nearest first linear portion 31d among the first linear portions 31 forming the wavy units 33 adjacent in the circumferential direction. The second linear portion 34 is disposed to be inclined at a predetermined angle with respect to the long-axis direction. An inclination angle of the second linear portion 34 with respect to the long-axis direction is larger than inclination angles of the first linear portion 31a and the first linear portion 31d with respect to the long-axis direction.

The second bent portion 35 is connected to the distal end side or the proximal end side of the second linear portion 34 on one end side, and is connected to the nearest first linear portion 31d in the wavy units 33 adjacent in the circumferential direction on the other end side. As illustrated in Fig. 3, the second bent portions 35 include: a second bent portion 35a connecting the first linear portion 31a and the second linear portion 34 of another basic unit 36 adjacent in the circumferential direction; a second bent portion 35b connecting the first linear portion 31d and the second linear portion 34; and a second bent portion 35c connecting the second linear portion 34 and the first linear portion 31a of still another basic unit 36 adjacent in the circumferential direction.

The strut forming the annular member 10 is formed by continuously disposing a plurality of the basic units 36 in the circumferential direction. In the present embodiment, the strut is formed by connecting four basic units 36 as illustrated in Fig. 2. That is, the annular member 10 is formed of the four basic units 36.

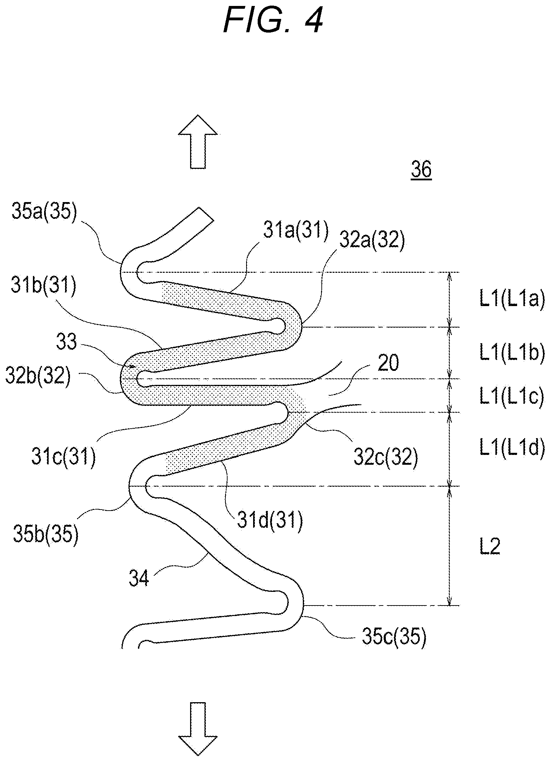

As illustrated in Fig. 3, the basic unit 36 is formed such that a second distance L2, which is a separation distance in the circumferential direction between an extreme end portion in the long-axis direction of the second bent portion 35 connected to an end portion on one end side of the second linear portion 34 and an extreme end portion in the long-axis direction of the second bent portion 35 connected to an end portion on the other end side of the second linear portion 34, is longer than a first distance L1 which is a separation distance in the circumferential direction between an extreme end portion in the long-axis direction of the first bent portion 32 connected to an end portion on one end side of any of the first linear portions 31 and an extreme end portion in the long-axis direction of the first bent portion 32 or the second bent portion 35 connected to an end portion on the other end side of the first linear portion 31. As illustrated in Figs. 3 and 4, the relationship between the first distance L1 and the second distance L2 is maintained even before and after expansion of the stent 100.

The annular members 10 adjacent to each other in the long-axis direction are integrally connected by the link portion 20. The adjacent annular members 10 are connected by at least one or more link portions 20 in a gap D between the annular members 10. In the present embodiment, the adjacent annular members 10 are connected by two link portions 20.

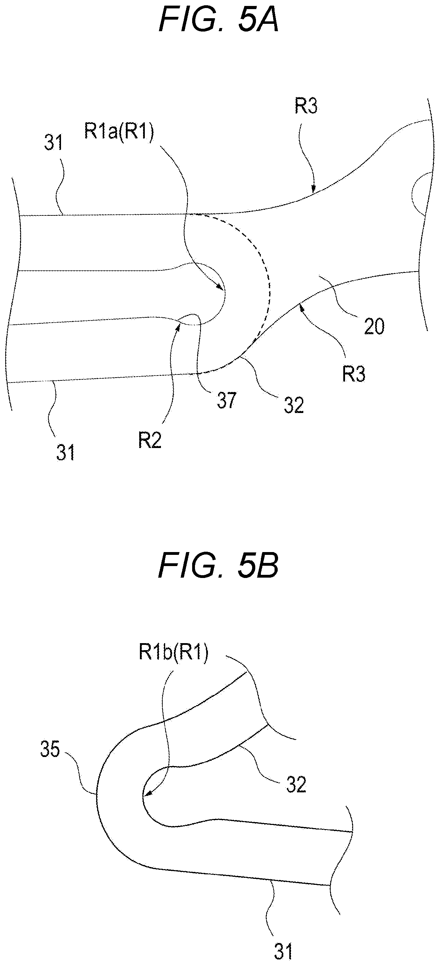

A distal end portion of the link portion 20 is connected to the first bent portion 32c of the annular member 10 located on the distal end side. Furthermore, a proximal end portion of the link portion 20 is connected to the first bent portion 32b of the annular member 10 located on the proximal end side. The first linear portion 31b and the first linear portion 31c, which is the axially parallel linear portion, are connected to the first bent portion 32b connected to the link portion 20. The first linear portion 31c, which is the axially parallel linear portion, and the first linear portion 31d are connected to the first bent portion 32c connected to the link portion 20. Note that the first bent portion 32c connected to the link portion 20 is a bent region that connects a proximal end of the first linear portion 31c and a proximal end of the first linear portion 31d. An outer curved line of the first bent portion 32c, that is, a boundary line between the link portion 20 and the first bent portion 32c is an arc inscribed in an edge line of the first linear portion 31c closer to the first linear portion 31b and an edge line of the first linear portion 31d closer to the second linear portion 34 as illustrated in Fig. 5A. The first bent portion 32b also has a bent region and an outer curved line (not illustrated) similar to those of the first bent portion 32c.

Examples of a material forming the strut include metal materials such as stainless steel, a cobalt-based alloy such as a cobalt-chromium alloy (for example, a CoCrWNi alloy), a platinum-chromium alloy (for example, a PtFeCrNi alloy), and a nickel-titanium alloy, and biodegradable polymer materials such as polylactic acid, polyglycolic acid, a lactic acid-glycolic acid copolymer, polycaprolactone, a lactic acid-caprolactone copolymer, and a glycolic acid-caprolactone copolymer.

A covering member containing a drug may be provided on an outer surface of the strut. The covering member is preferably formed on an outer surface located on the radially outer side out of the outer surface of the strut, but is not limited thereto. The covering member may contain the drug capable of suppressing proliferation of the neointima and a drug carrier for carrying the drug. The covering member may be configured to contain only the drug. The drug in the covering member is, for example, at least one kind selected from the group consisting of sirolimus, everolimus, zotarolimus, paclitaxel, and the like. A constituent material of the drug carrier is not particularly limited, but is preferably a biodegradable material.

The stent 100 of the present embodiment is formed such that the second distance L2 is longer than the first distance L1. For this reason, when the stent 100 is pressure-crimped to the balloon 220, the balloon 220 is less likely to protrude from a region between the first linear portions 31 adjacent in the circumferential direction, but is likely to protrude from a region between the first linear portion 31 and the second linear portion 34 adjacent in the circumferential direction, and thus, a stent-retaining force with respect to the balloon 220 is stabilized. Furthermore, the stent 100 of the present embodiment has four or more first linear portions 31 constituting the wavy unit 33. For this reason, a shape of the annular member 10 when being enlarged to a larger diameter is more wavy as compared with that in a stent in which the number of the first linear portions 31 is less than four, and a local radial force becomes uniform.

In the stent 100 of the present embodiment, the number of first linear portions 31 forming the wavy units 33 is four or more and is an even number. Furthermore, the number of the second linear portions 34 forming the basic unit 36 is one. Since such a configuration is adopted, in the stent 100, inclined postures with respect to the long-axis direction from the proximal end side to the distal end side of the second linear portions 34 in the basic units 36 adjacent in the circumferential direction are opposite (form a substantially V-shape), and a design of the annular member 10 becomes point-symmetric. Therefore, in one basic unit 36 of the annular member 10 of the stent 100, bent portions that are likely to open or less likely to open are not concentrated on one end portion in the annular member 10 even when the bent portions that are likely to open or are less likely to open at the time of enlargement are concentrated on the proximal end side, or conversely, the bent portions that are less likely to open or are likely to open at the time of enlargement are concentrated on the distal end side, and thus, the expansion uniformity is improved.

In the stent 100 of the present embodiment, the number of the plurality of first linear portions 31 forming the wavy unit 33 is four. Since such a configuration is adopted, the stent 100 can satisfy the stabilization of the stent-retaining force and the uniformity of the local radial force, and be smoothly sent to a lesion at a peripheral site since an outer diameter (profile) at the time of reduction when being mounted on the balloon 220 is reduced.

Furthermore, the stent 100 of the present embodiment is formed such that a line width of the first bent portion 32 is smaller than a line width of the first linear portion 31 connected thereto. Furthermore, a line width of the second bent portion 35 is formed to be smaller than a line width of the second linear portion 34 connected thereto.

Typically, when the stent 100 is expanded and deformed, tensile stress acts on an inner curved side and compressive stress acts on an outer curved side of each of the "bent portions" including the first bent portion 32 and the second bent portion 35, the vicinity of the center of the line width is a neutral surface, and the magnitude of each stress is proportional to a distance from the neutral surface. Therefore, when a stent including a bent portion having a smaller line width and a stent including a bent portion having a larger line width are expanded to the same diameter, the tensile stress acting on the inner curved side is smaller in the stent including the bent portion having the smaller line width than that in the stent including the bent portion having the larger line width. Furthermore, when the stent is expanded and deformed to an expansion limit diameter at which breakage occurs, the expansion limit diameter is larger in the stent including the bent portion having the smaller line width than that in the stent including the bent portion having the larger line width. Therefore, in a case where the line width of the bent portion is smaller than the line width of the linear portion in the linear portions and the bent portions that are connected as in the stent 100 of the present embodiment, the tensile stress acting on the inner curved side of the bent portion is smaller under a condition that open states of the bent portions are the same, that is, under a condition that angles formed by the linear portion connected to one end of the bent portion and the linear portion connected to the other end are the same as compared with a case where the line width of the bent portion is the same as the line width of the linear portion, and thus, risk of stent breakage can be mitigated.

Furthermore, the tensile stress and the expansion limit diameter on the inner curved side of the bent portion also change depending on a radius of curvature on the inner curved side of the bent portion. As the radius of curvature on the inner curved side increases, the tensile stress acting on the inner curved side of the bent portion when deformation providing the same expansion diameter is applied to the bent portion decreases, and the expansion limit diameter increases. In a case where a bent portion having a larger radius of curvature on the inner curved side and a bent portion having a smaller radius of curvature on the inner curved side are present in one stent, the bent portion having the larger radius of curvature on the inner curved side is more likely to open at the time of expansion. Therefore, when the radius of curvature on the inner curved side of each of the bent portions in the stent is adjusted, the ease of opening of each of the bent portions changes, and the expansion uniformity, which means the degree of distribution of the open states of the bent portions, changes.

As illustrated in Figs. 5A and 5B, the stent 100 of the present embodiment is formed such that a second radius R1b, which is a radius of curvature on the inner curved side of the second bent portion 35, is larger than a first radius R1a, which is a radius of curvature on the inner curved side of the first bent portion 32, out of radii R1 of curvature on the inner curved side of the bent portions. Since such a configuration is adopted, the stent 100 can reduce the risk of stent breakage.

The stent 100 of the present embodiment is formed such that the radius of curvature on the inner curved side of the first bent portion 32 to which the link portion 20 is connected is larger than the radius of curvature on the inner curved side of the first bent portion 32 to which the link portion 20 is not connected. Since such a configuration is adopted, the first bent portion 32 connected to the link portion 20 is more likely to open in the stent 100 as compared with a case where a radius of curvature on the inner curved side of each of the first bent portions 32 is the same, and the expansion uniformity is also improved.

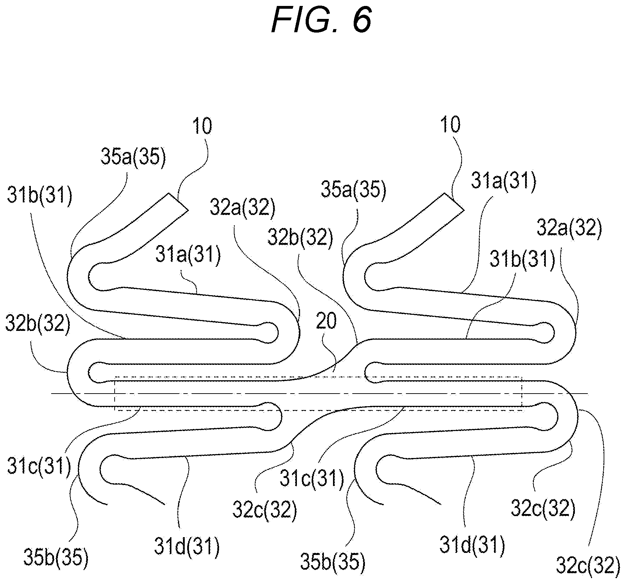

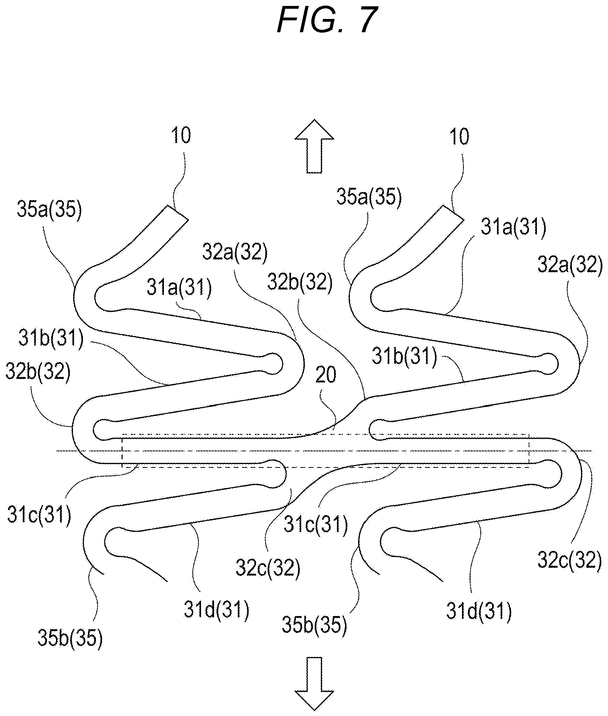

As illustrated in Figs. 6 and 7, in the stent 100 of the present embodiment, a portion from the proximal end side of the first linear portion 31c that becomes the axially parallel linear portion of the annular member 10 on the proximal end side to the distal end side of the first linear portion 31c that becomes the axially parallel linear portion of the annular member 10 on the distal end side via the link portion 20 (a portion surrounded by a dotted-line region in the drawings) is parallel to the long-axis direction before and after the stent expansion. Since such a configuration is adopted, it is possible to prevent a decrease (shortening) of a length of the stent 100 in the long-axis direction when the stent is expanded.

In the stent 100 of the present embodiment, the link portions 20 are disposed in a pair in each of the gaps D between the adjacent annular members 10 so as to face each other in the radial direction of the annular member 10, and disposed such that a phase of the link portion 20 disposed in one gap D and a phase of the link portion 20 disposed in another gap D adjacent to the one gap D in the long-axis direction are shifted by 90° in the circumferential direction. Since such a configuration is adopted, the link portion 20 of the another adjacent gap D is present at an intermediate position in the circumferential direction of the link portions 20 adjacent in the circumferential direction provided in the one gap D, and circumferential positions where the link portions 20 are placed are dispersed with respect to the overall length of the stent in the stent 100, and thus, the flexibility at the circumferential position of the stent becomes more uniform.

Here, specific dimensional examples of the stent 100 will be described. The stent 100 to be actually manufactured is sometimes slightly different from exemplified values due to variations at the time of manufacturing. The overall length of the stent 100 in the long-axis direction is 5 mm to 200 mm, preferably 9 mm to 50 mm. An outer diameter of the stent 100 at the time of crimping is 0.5 mm to 2 mm. An expansion diameter of the stent 100 is 3.0 mm to 6.5 mm, preferably 3.5 mm to 4.5 mm. A thickness of the strut is 40 um to 150 µm.

A length (a distance between extreme end portions in the long-axis direction of the bent portions adjacent in the circumferential direction, hereinafter, also referred to as an "amplitude") of the annular member 10 in the long-axis direction is 500 µm to 1500 um. The number of the annular members 10 is 4 to 80, preferably 8 to 40.

The first distance L1 in the basic unit 36 is 50 um to 500 um, preferably 100 um to 300 um in a state before being crimped to the balloon 220. The first distance L1 in the basic unit 36 is 50 um to 500 um, preferably 100 um to 200 um in a state after being crimped to the balloon 220. The first distance L1 in the basic unit 36 is 200 um to 1200 um, preferably 200 um to 900 um in a state where the stent 100 crimped to the balloon 220 has been expanded until a stent inner diameter reaches 4.0 mm, and then the balloon 220 has been removed. The first distance L1 in the basic unit 36 is 200 um to 1200 um, preferably 300 um to 1000 um in a state where the stent 100 crimped to the balloon 220 has been expanded until the stent inner diameter reaches 4.18 mm, and then the balloon 220 has been removed.

The second distance L2 in the basic unit 36 is 500 um to 1000 um, preferably 200 um to 900 um in the state before being crimped to the balloon 220. The second distance L2 in the basic unit 36 is 100 um to 1000 um, preferably 200 um to 500 um in the state after being crimped to the balloon 220. The second distance L2 in the basic unit 36 is 200 µm to 1500 um, preferably 500 um to 1200 um in the state where the stent 100 crimped to the balloon 220 has been expanded until the stent inner diameter reaches 4.0 mm, and then the balloon 220 has been removed. The second distance L2 in the basic unit 36 is 200 µm to 1500 um, preferably 500 um to 1200 um in the state where the stent 100 crimped to the balloon 220 has been expanded until the stent inner diameter reaches 4.18 mm, and then the balloon 220 has been removed.

A length of the link portion 20 in the long-axis direction, that is, an axial length between an extreme end portion on the proximal end side of the first bent portion 32c connected to the link portion 20 and an extreme end portion on the distal end side of the first bent portion 32b connected to the link portion 20 is 200 um to 600 um. A minimum line width of the link portion 20 is 50 um to 400 um. A maximum line width of the link portion 20 is 200 um to 500 µm.

A line width of the first linear portion 31 is 50 um to 200 um. A line width of the first bent portion 32 is 50 um to 200 um. A line width of the second linear portion 34 is 50 um to 200 um. A line width of the second bent portion 35 is 50 um to 200 µm.

The radius of curvature on the inner curved side (first radius R1a) of the first bent portion 32 and the radius of curvature on the inner curved side (second radius R1b) of the second bent portion 35 are 20 um to 120 um. Among them, a radius of curvature on the inner curved side of the first bent portion 32 not connected to the link portion 20 is preferably 20 um to 90 um, and a radius of curvature on the inner curved side of the first bent portion 32 connected to the link portion 20 is preferably 50 um to 120 um. Furthermore, the radius of curvature on the inner curved side of the second bent portion 35 is preferably 50 um to 120 µm.

As illustrated in Fig. 5A, a radius R2 of curvature on the inner curved side of a connection portion 37, which is a connection portion between the first bent portion 32 (or the second bent portion 35) and the first linear portion 31 (or the second linear portion 34), is 50 um to 500 um, preferably 100 um to 300 um. A radius R3 of curvature of an outer edge of the link portion 20 is 200 um to 2000 um, preferably 300 um to 800 µm.

### [Operational Effects]

As described above, the stent 100 according to the present embodiment is formed in an annular shape by a linear component folded in a wave shape and includes the plurality of annular members 10 disposed along the long-axis direction of the cylindrical shape so as to form the cylindrical shape, and the link portion 20 connecting the adjacent annular members 10, and is configured to be expandable and contractible in the radial direction of the cylindrical shape. Furthermore, in the stent 100 having such a configuration, the annular member 10 is formed by disposing the plurality of basic units 36 in the circumferential direction and connecting the adjacent basic units 36 by the second bent portion 35, the basic unit 36 including: the wavy unit 33, which is formed of the plurality of first linear portions 31 extending from the proximal end side to the distal end side in the long-axis direction and disposed continuously in the circumferential direction of the cylindrical shape and the first bent portion 32 connecting the end portions on the proximal end side or the distal end side of two first linear portions 31 adjacent in the circumferential direction; the second linear portion 34 extending from the proximal end side to the distal end side in the long-axis direction and disposed at the position adjacent to the first linear portion 31 in the circumferential direction; and the second bent portion 35 connecting the end portions on the proximal end side or the distal end side of the first linear portion 31 and the second linear portion 34 adjacent in the circumferential direction. Furthermore, in the stent 100, the second distance L2, which is the separation distance in the circumferential direction between the extreme end portion in the long-axis direction of the second bent portion 35 connected to the end portion on one end side of the second linear portion 34 and the extreme end portion in the long-axis direction of the second bent portion 35 connected to the end portion on the other end side of the second linear portion 34, is longer than the first distance L1 which is the separation distance in the circumferential direction between the extreme end portion in the long-axis direction of the first bent portion 32 connected to the end portion on one end side of any of the first linear portions 31 and the extreme end portion in the long-axis direction of the first bent portion 32 or the second bent portion 35 connected to the end portion on the other end side of the first linear portion 31, and the number of the first linear portions 31 disposed in the wavy unit 33 is four or more.

With such a configuration, when the stent 100 is pressure-crimped to the balloon 220, the balloon 220 is less likely to protrude from the region between the first linear portions 31 adjacent in the circumferential direction, but is likely to protrude from the region between the first linear portion 31 and the second linear portion 34 adjacent in the circumferential direction, so that the stent-retaining force with respect to the balloon 220 is stabilized. Furthermore, in the stent 100, the shape of the annular member 10 when being enlarged to a larger diameter is more wavy as compared with that in a stent in which the number of the first linear portions 31 is less than four, and the local radial force becomes uniform.

Furthermore, in the basic unit 36 of the stent 100 according to the present embodiment, the number of the first linear portions 31 may be an even number, and the number of the second linear portions 34 may be one.

With such a configuration, in the stent 100, inclinations with respect to the long-axis direction from the proximal end side to the distal end side of the second linear portions 34 in the basic units 36 adjacent in the circumferential direction are opposite (form a substantially V-shape), and the design of the annular member 10 becomes point-symmetric. Therefore, in one basic unit 36 of the annular member 10 of the stent 100, bent portions that are likely to open or less likely to open are not concentrated on one end portion in the annular member 10 even when the bent portions that are likely to open or are less likely to open at the time of enlargement are concentrated on the proximal end side, or conversely, the bent portions that are less likely to open or are likely to open at the time of enlargement are concentrated on the distal end side, and thus, the expansion uniformity is improved.

Furthermore, the number of the first linear portions 31 may be four in the stent 100 according to the present embodiment.

With such a configuration, the stent 100 can satisfy the stabilization of the stent-retaining force and the uniformity of the local radial force, and be smoothly sent to a lesion at a peripheral site since an outer diameter (profile) at the time of reduction when being mounted on the balloon 220 is reduced.

Furthermore, the line width of the first bent portion 32 may be smaller than the line width of the first linear portion 31 connected thereto in the stent 100 according to the present embodiment.

With such a configuration, the tensile stress acting on the inner curved side of the first bent portion 32 when deformation providing the same expansion diameter is applied to the first bent portion 32 decreases as compared with a case where the line width of the first bent portion 32 is the same as the line width of the first linear portion 31, and thus, the risk of stent breakage can be reduced in the stent 100.

Furthermore, the line width of the second bent portion 35 may be smaller than the line widths of the first linear portion 31 and the second linear portion 34 connected thereto in the stent 100 according to the present embodiment.

With such a configuration, the tensile stress acting on the inner curved side of the second bent portion 35 when deformation providing the same expansion diameter is applied to the second bent portion 35 decreases as compared with a case where the line width of the second bent portion 35 is the same as the line widths of the first linear portion 31 and the second linear portion 34, and thus, the risk of stent breakage can be reduced in the stent 100. Furthermore, the second bent portion 35 is more likely to open at the time of stent expansion as compared with the case where the line width of the second bent portion 35 is the same as the line widths of the first linear portion 31 and the second linear portion 34, and the expansion uniformity is improved.

Furthermore, the second radius R1b, which is the radius of curvature on the inner curved side of the second bent portion 35, may be larger than the first radius R1a, which is the radius of curvature on the inner curved side of the first bent portion 32, in the stent 100 according to the present embodiment.

With such a configuration, the stent 100 can reduce the risk of stent breakage. Furthermore, the second bent portion 35 is more likely to open than the first bent portion 32, and the expansion uniformity is improved.

Furthermore, in the stent 100 according to the present embodiment, the radius of curvature on the inner curved side of the first bent portion 32c to which the link portion 20 is connected may be larger than the radius of curvature on the inner curved side of each of the first bent portions 32a and 32b to which the link portion 20 is not connected.

With such a configuration, the first bent portion 32 connected to the link portion 20 is more likely to open in the stent 100 as compared with the case where the radius of curvature on the inner curved side of each of the first bent portions 32 is the same, and the expansion uniformity is also improved.

Furthermore, in the stent 100 according to the present embodiment, the first linear portion 31 may include the first linear portions 31b and 31c which are the axially parallel linear portions parallel to the long-axis direction, the phases of the plurality of annular members 10 in the circumferential direction may be aligned, the link portion 20 may connect the first bent portion 32 connected to the distal end side of the axially parallel linear portion of the annular member 10 disposed on the proximal end side and the first bent portion 32 connected to the proximal end side of the axially parallel linear portion of the annular member 10 disposed on the distal end side adjacent to the annular member 10 disposed on the proximal end side, and a portion from the proximal end side of the axially parallel linear portion of the annular member 10 on the proximal end side to the distal end side of the axially parallel linear portion of the annular member 10 on the distal end side via the link portion 20 may be parallel to the long-axis direction before and after expansion of the stent 100.

With such a configuration, shortening at the time of the stent expansion can be prevented.

Furthermore, in the stent 100 according to the present embodiment, two link portions 20 may be disposed in each of the gaps D between the adjacent annular members 10 so as to face each other in the radial direction of the annular member 10, and the phase of the link portion 20 disposed in one gap D and the phase of the link portion 20 disposed in another gap D adjacent to the one gap D in the long-axis direction may be shifted by 90° in the circumferential direction.

With such a configuration, the link portion 20 of the another adjacent gap D is present at an intermediate position in the circumferential direction of the link portions 20 adjacent in the circumferential direction provided in the one gap D, and circumferential positions where the link portions 20 are placed are dispersed with respect to the overall length of the stent in the stent 100, and thus, the flexibility at the circumferential position of the stent becomes more uniform.

Furthermore, the stent delivery system 300 according to the present embodiment includes the stent 100 described any one of the above, and the balloon catheter 200 including the inflatable and deflatable balloon 220. The stent 100 is retained in close contact with the deflated balloon 220 in a contracted state, and the balloon 220 protrudes outward from a radial position of an inner surface of the stent 100 only at a position between the first linear portion 31 and the second linear portion 34.

With such a configuration, the stent 100 has a more stabilized stent-retaining force with respect to the balloon 220 and can be more reliably sent to a target lesion.

### [Modifications]

Note that the above-described embodiment can also be appropriately changed according to a use environment and the like as described below. Furthermore, the following changes can also be implemented in any combination without departing from the gist of the present invention.

The second linear portion 34 has a curved shape in the stent 100 of the present embodiment described above, but is not limited to this shape, and may have a straight shape. Furthermore, the first linear portion 31 has a substantially straight shape, but is not limited to this shape, and may have a curved shape.

Furthermore, the connection portion 37 having a curved shape is provided between the first linear portion 31 or the second linear portion 34 and the first bent portion 32 or the second bent portion 35 in the stent 100 of the present embodiment, but the connection portion 37 may have a straight shape. The first linear portion 31 or the second linear portion 34 may be connected to the first bent portion 32 or the second bent portion 35 without providing the connection portion 37.

Furthermore, at least a part of curves on the inner curved side and/or the outer curved side of the first bent portions 32, the second bent portions 35, and the link portions 20 may be formed to be a continuation of a plurality of arcs.

Furthermore, a line width of at least a part of the first bent portions 32 or the second bent portions 35 may be constant, or may gradually decrease from one end side to the other end side, or from an intermediate portion to an end portion. Alternatively, a line width of at least a part of the first linear portions 31 or the second linear portions 34 may be constant, or may gradually decrease from one end side to the other end side, or from an intermediate portion to an end portion.

### Examples

Hereinafter, the present invention will be specifically described with reference to Examples, but the scope of the present invention is not limited to the following Examples.

### [Evaluation Test 1]

In Evaluation Test 1, stents were actually produced, and the first distance L1 and the second distance L2 at each stent diameter when the stent diameter was changed were measured. A stent was produced by preparing a drawing in which a stent design is drawn, irradiating a pipe made of a CoCr alloy having an outer diameter of 2 mm with a laser beam on the basis of the drawing to cut out the stent design, performing polishing, and then performing a heat treatment. In this state before pressure crimping, the first distance L1 and the second distance L2 were measured. This stent was mounted to a balloon of a balloon catheter by a pressure crimping method, and then, the first distance L1 and the second distance L2 were measured. Thereafter, the balloon and the stent were expanded at 11 atm of the balloon. Thereafter, the balloon was depressurized to remove the balloon catheter from the stent. Thereafter, the first distance L1 and the second distance L2 were measured. Furthermore, another stent was similarly produced and similarly mounted to a balloon. Thereafter, the balloon and the stent were expanded at 16 atm of the balloon. Thereafter, the balloon was depressurized to remove a balloon catheter from the stent, and then, the first distance L1 and the second distance L2 were measured. Note that the first distance L1 and the second distance L2 at each point in time were measured together with a stent outer diameter.

The balloons used in Evaluation Test 1 had balloon diameters of 4.0 mm and 4.18 mm under the pressures of 11 atm and 16 atm, respectively. The stent outer diameters and the first distance L1 and the second distance L2 at each of the stent outer diameters were measured using a digital microscope "VHX-5000 (manufactured by KEYENCE CORPORATION)" and measured as distances projected on a plane. Note that the distance projected on the plane has a unique transformation relationship with a distance along the circumferential direction in terms of geometry, and thus, the magnitude relationship when comparing a plurality of distances is the same regardless of which distance is selected and compared.

Dimensional values of the stent design prepared in the drawing are as follows. The drawing uses drafting software that can be used on a computer, and the dimensional values are values on the drafting software. Furthermore, the dimensional values are target values of dimensions after the polishing when the stent is actually produced, but dimensional values of the actually produced stent become values deviating from the target values due to manufacturing variations.

### <Dimension Value>

- Outer diameter: 2.00 mm
- First distance L1a between second bent portion 35a and first bent portion 32a: 260 um
- First distance L1b between first bent portion 32a and first bent portion 32b: 185 um
- First distance L1c between first bent portion 32b and first bent portion 32c: 200 um
- First distance L1d between first bent portion 32c and second bent portion 35b: 245 um
- Second distance L2 between second bent portion 35b and second bent portion 35c: 680 um
- Amplitude between second bent portion 35a and first bent portion 32a: 1065 um
- Amplitude between first bent portion 32a and first bent portion 32b: 1065 um
- Amplitude between first bent portion 32b and first bent portion 32c: 1080 um
- Amplitude between first bent portion 32c and second bent portion 35b: 1030 um
- Amplitude between second bent portion 35b and second bent portion 35c: 1030 um
- Amplitude between second bent portion 35a and first bent portion 32c or second bent portion 35c that corresponds to amplitude of basic unit 36: 1080 um
- Length of link portion 20 in long-axis direction: 230 um
- Minimum line width of link portion 20: 183 um
- Maximum line width of link portion 20: 290 um
- Line width of each of first linear portion 31 and second linear portion 34: 120 um
- Line width of each of first bent portion 32 and second bent portion 35: 95 um
- Radius R1 of curvature on inner curved side of each of second bent portions 35a, 35b, and 35c: 70 um
- Radius R2 of curvature on inner curved side of connection portion 37 connected between second bent portion 35a and first linear portion 31a: 100 um
- Radius R1 of curvature on inner curved side of the first bent portion 32a: 50 µm
- Radius R2 of curvature on inner curved side of connection portion 37 connected between first bent portion 32a and first linear portion 31a: 100 µm
- Radius R1 of curvature on inner curved side of the first bent portion 32b: 50 µm
- Radius R2 of curvature on inner curved side of connection portion 37 connected between first bent portion 32b and first linear portion 31b: 100 um
- Radius R1 of curvature on inner curved side of first bent portion 32c: 65 um
- Radius R2 of curvature on inner curved side of connection portion 37 connected between first bent portion 32c and first linear portion 31c: 100 µm
- Radius R3 of curvature of outer edge of link portion 20: 400 µm
- Thickness of strut: 85 um

**[Table 1]**

| | Stent outer diameter (mm) | Region A | | | | | Region B | | | | |
|---|---|---|---|---|---|---|---|---|---|---|---|
| | | L1a [µm] | L1b [µm] | L1c [µm] | L1d [µm] | L2 [µm] | L1a [µm] | L1b [µm] | L1c [µm] | L1d [µm] | L2 [µm] |
| Before crimping | 1.95 | 263 | 174 | 191 | 231 | 657 | 265 | 178 | 196 | 238 | 649 |
| After crimping | 1.21 | 152 | 159 | 154 | 170 | 342 | 164 | 142 | 171 | 151 | 260 |
| After expansion (4 atm) | 3.96 | 628 | 565 | 302 | 373 | 748 | 791 | 697 | 501 | 567 | 934 |
| After expansion (16 atm) | 4.09 | 656 | 627 | 332 | 436 | 873 | 840 | 738 | 562 | 590 | 958 |

### <Evaluation Results>

Test results of Evaluation Test 1 were as shown in Table 1 above. In Table 1, "Region A" indicates a region A surrounded by a one-dot chain line in Fig. 2, and "Region B" indicates a region B surrounded by a one-dot chain line in Fig. 2. That is, the region A and the region B are regions including the basic units 36, respectively, disposed at positions facing each other in the radial direction in one annular member 10.

As shown in Table 1, the stent outer diameters at the points in time of before the pressure crimping, after the pressure crimping, after depressurization after the balloon has been inflated at 11 atm, and after depressurization after the balloon has been inflated at 16 atm were 1.95 mm, 1.21 mm, 3.96 mm, and 4.09 mm, respectively. For any of these stent outer diameters, the relationship between the first distance L1 (L1a to L1d) and the second distance L2 was L1 < L2.

Based on the above results, the following contents are supplemented regarding the stent according to the present embodiment. The stent according to the present embodiment has a feature that the second distance L2 is longer than the first distance L1, but this is not limited to a specific stent diameter. Therefore, a stent described in the claims of the present invention is not interpreted as being limited to a specific stent diameter, and stents each having a stent diameter in which the second distance L2 is longer than the first distance L1 are included in the stent described in the claims of the present invention. Note that the first distance L1 and the second distance L2 are affected by the distribution of an external force applied by a diameter reduction operation or an enlargement operation. Since the distribution varies every time these operations are performed, the first distance L1 and the second distance L2 vary greatly at a stent diameter obtained after these operations. Therefore, the first distance L1 is sometimes longer than the second distance L2 within a variation range even in the stent described in the claims of the present invention. However, if the second distance L2 is longer than the first distance L1 at a certain stent diameter in terms of an average value between individuals, such a stent can contribute to stabilization of the stent-retaining force, and thus, such a stent also falls within the scope of the claims of the present invention.

### [Evaluation Test 2]

In Evaluation Test 2, stent designs (Examples 1 to 13 in Table 2) obtained by appropriately changing values of R1 to R3 of the first bent portion 32, the second bent portion 35, and the link portion 20 based on the stent design prepared in Evaluation Test 1 were modeled on FEM analysis application software "Abaqus (manufactured by Dassault Systems SE)", and expansion limit diameters and expansion distances shown below were analyzed at the time of stent expansion.

The "expansion limit diameter" is a diameter at the time of occurrence of a position where a maximum value of a tensile strain reaches a predetermined value (0.6) when the stent model is expanded. In Examples 1 to 13, a breaking point of a CoCr alloy as a constituent material is about 0.6, and thus, this diameter value serves as an indicator of a diameter at which stent breakage occurs. The larger diameter value indicates that the stent is less likely to break.

The "expansion distance" is a distance in the circumferential direction between an extreme end portion in the long-axis direction of the first bent portion 32b and an extreme end portion in the long-axis direction of the second bent portion 35b. From the viewpoint of expansion uniformity, if distances in the circumferential direction between bent portions adjacent in the circumferential direction present in the annular member are all equal at the time of stent expansion, it means that its expansion state is uniform. When the number of the bent portions in the annular member 10 is "10" and a stent inner diameter is 3.5 mm, the distance is calculated to be 1.10 mm. Therefore, in this case, the extension distance being closer to 1.10 mm indicates the higher extension uniformity.

Conditions used for performing this analysis are shown as below.
- Material properties: A material having a correlation between true stress and a true strain obtained by a tensile test of a CoCr alloy as a raw material was used.
- Element properties: An elastoplastic member was used. Element type: C3D8R + M3D4M obtained by wrapping a three-dimensional reduction element on a three-dimensional reduction integration element was used.
- Boundary condition: A cylindrical part (A) having an inner diameter larger than a stent outer diameter was placed around the stent and the part (A) was contracted until the stent outer diameter reached 1.2 mm. Thereafter, this part (A) was removed, a cylindrical part (B) having an outer diameter smaller than the stent inner diameter was placed inside the contracted stent, and the part (B) was expanded until the stent inner diameter reached 3.5 mm. Material properties of the parts (A) and (B) were set to superelastic properties.

**[Table 2]**

| | Geometry | | | | | | | | | Analysis results | |
|---|---|---|---|---|---|---|---|---|---|---|---|
| | Second bent portion | | First bent portion A | | First bent portion B | | First bent portion C | | Link portion | Expansion limit diameter [mm] | Expansion distance [mm] |
| | R1 [µm] | R2 [µm] | R1 [µm] | R2 [µm] | R1 [µm] | R2 [µm] | R1 [µm] | R2 [µm] | R3 [µm] | | |
| Example 1 | 50 | 100 | 50 | 100 | 50 | 100 | 50 | 100 | 690 | 3.71 | 0.9 |
| Example 2 | 70 | 100 | 50 | 100 | 50 | 100 | 50 | 100 | 690 | 5.59 | 0.8 |
| Example 3 | 70 | 100 | 50 | 100 | 50 | 100 | 50 | 100 | 400 | 5.63 | 0.84 |
| Example 4 | 70 | 100 | 50 | 100 | 50 | 100 | 55 | 100 | 400 | 5.82 | 0.92 |
| Example 5 | 70 | 100 | 50 | 100 | 50 | 100 | 65 | 100 | 400 | 5.87 | 0.97 |
| Example 6 | 50 | 300 | 50 | 300 | 50 | 300 | 50 | 300 | 690 | 3.81 | 0.88 |
| Example 7 | 70 | 300 | 50 | 300 | 50 | 300 | 50 | 300 | 690 | 5.62 | 0.79 |
| Example 8 | 70 | 300 | 50 | 300 | 50 | 300 | 50 | 300 | 400 | 5.63 | 0.84 |
| Example 9 | 70 | 100 | 50 | 300 | 50 | 300 | 50 | 300 | 690 | 5.63 | 0.78 |
| Example 10 | 70 | 300 | 50 | 100 | 50 | 100 | 50 | 100 | 690 | 5.59 | 0.81 |
| Example 11 | 70 | 300 | 50 | 300 | 50 | 300 | 40 | - | 400 | 5.55 | 0.73 |
| Example 12 | 70 | 300 | 50 | 300 | 65 | 300 | 40 | - | 400 | 5.76 | 0.76 |
| Example 13 | 70 | 300 | 50 | 300 | 65 | 300 | 40 | - | 690 | 5.73 | 0.75 |

| | | | | | | | | | | | |
|---|---|---|---|---|---|---|---|---|---|---|---|
| * -: Connection portion is not provided | | | | | | | | | | | |

### <Evaluation Results>

Test results of Evaluation Test 2 were as shown in Table 2 above. In Table 2, "Second Bent Portion" corresponds to all of the second bent portions 35a, 35b, and 35c, "first bent portion A" corresponds to the first bent portion 32a, "First Bent Portion B" corresponds to the first bent portion 32b, and "First Bent Portion C" corresponds to the first bent portion 32c connected to the link portion 20. In Table 2, the units of R1 to R3 are all "um".

As shown in Table 2, in the stent 100 of Example 1, the radius R1 (first radius R1a) of curvature on the inner curved side of the first bent portion 32 and the radius R1 (second radius R1b) of curvature on the inner curved side of the second bent portion 35 have the same value. In the stent 100 of Example 2, the second radius R1b, which is the radius of curvature on the inner curved side of the second bent portion 35, is larger than the first radius R1a which is the radius of curvature on the inner curved side of the first bent portion 32. When comparing the stent 100 of Example 1 with the stent 100 of Example 2, the expansion limit diameter was larger in the stent 100 of Example 2. Since the second radius R1b is larger than the first radius R1a in the stent 100 of Example 2, it is presumed that concentration of stress on the second bent portion 35 at the time of stent expansion has been mitigated, and the expansion limit diameter has increased. Furthermore, similar results are also understood from a comparison between a stent of Example 6 and a stent of Example 7. As described above, the risk of stent breakage can be reduced since the second radius R1b is larger than the first radius R1a in the stent 100 according to the present embodiment.

As shown in Table 2, the radius R1 of curvature on the inner curved side of the first bent portion C of the stent 100 of Example 3 is smaller than the radius R1 of curvature on the inner curved side of the first bent portion C of the stent 100 of Example 4. The radius R1 of curvature on the inner curved side of the first bent portion C of the stent 100 of Example 4 is smaller than the radius R1 of curvature on the inner curved side of the first bent portion C of the stent 100 of Example 5. When comparing the stents 100 of Example 3, Example 4, and Example 5, the expansion distance has increased as the radius R1 of curvature on the inner curved side of the first bent portion C increases. The first bent portion C is connected to the link portion 20, and thus, is less likely to open than the other first bent portions A and B, but it is presumed that the first bent portion C has been more likely to open as the radius of curvature of the first bent portion C becomes larger than the radii of curvature of the first bent portions A and B. As described above, the first bent portion 32 connected to the link portion 20 is more likely to open than the first bent portion 32 not connected to the link portion 20 since the radius of curvature on the inner curved side of the first bent portion 32 to which the link portion 20 is connected is larger than the radius of curvature on the inner curved side of the first bent portion 32 to which the link portion 20 is not connected in the stent 100 according to the present embodiment, and the expansion uniformity is also improved.

As shown in Table 2, the radius R2 of curvature on the inner curved side of the connection portion 37 of the stent 100 of Example 1 is 100 um, and the radius R2 of curvature on the inner curved side of the connection portion 37 of the stent 100 of Example 6 is 300 um. Furthermore, the radius R2 of curvature on the inner curved side of the connection portion 37 of the stent 100 of Example 2 is 100 um, and the radius R2 of curvature on the inner curved side of the connection portion 37 of the stent 100 of Example 7 is 300 um. As a result of comparing the stent 100 of Example 1 with the stent 100 of Example 6, and comparing the stent 100 of Example 2 with the stent 100 of Example 7, there was no large difference in the expansion limit diameter and the expansion distance.

As shown in Table 2, the radius R3 of curvature of an outer edge of the link portion 20 of the stent 100 of Example 2 was 690 um, and the radius R3 of curvature of an outer edge of the link portion 20 of the stent 100 of Example 3 was 400 um. Furthermore, the radius R3 of curvature of an outer edge of the link portion 20 of the stent 100 of Example 7 was 690 um, and the radius R3 of curvature of an outer edge of the link portion 20 of the stent 100 of Example 8 was 400 um. The expansion distance of the stent 100 of Example 2 was slightly smaller than the expansion distance of the stent 100 of Example 3, but there was no large difference. The expansion distance of the stent 100 of Example 7 was slightly smaller than the expansion distance of the stent 100 of Example 8, but there was no large difference.

In the stent 100 of Example 9 and the stent 100 of Example 10, the radii R1 of curvature on the inner side of the bent portions are equal, and the radii R2 of curvature on the inner side of the connection portions 37 connected to the respective bent portions are different from each other. As a result of comparing the stent 100 of Example 9 with the stent 100 of Example 10, there was no large difference in the expansion limit diameter and the expansion distance.

The radius R1 of curvature on the inner curved side of the first bent portion C of the stent 100 of Example 11, the radius R1 of curvature on the inner curved side of the first bent portion C of the stent 100 of Example 12, and the radius R1 of curvature on the inner curved side of the first bent portion C of the stent 100 of Example 13 are all smaller than the radius R1 of curvature on the inner curved side of each of the first bent portions A and B. The stents 100 of Examples 11, 12, and 13 had smaller expansion distances than the stents 100 of the other Examples. Since not only the first bent portion C is connected to the link portion 20 and is less likely to open than the other first bent portions A and B but also the radius of curvature of the first bent portion C is smaller than the radii of curvature of the first bent portions A and B, it is presumed that the first bent portion C is less likely to open, and the expansion uniformity decreases.

This application is based on Japanese Patent Application No. 2021-024885 filed on February 19, 2021, of which the contents are incorporated by reference in their entirety.

### Reference Signs List

10 annular member
20 link portion
31(31a to 31d) first linear portion
32(32a to 32c) first bent portion
33 wavy unit
34 second linear portion
35(35a to 35c) second bent portion
36 basic unit
37 connection portion
100 stent
200 balloon catheter
220 balloon
300 stent delivery system
R1 radius of curvature of bent portion (R1a first radius,
R1b second radius)

## Claims

1. A stent that is expandable and contractible in a radial direction of a cylindrical shape, the stent comprising:
a plurality of annular members formed in an annular shape by a linear component folded in a wave shape and disposed along a long-axis direction of the cylindrical shape to form the cylindrical shape; and
a link portion connecting the annular members adjacent to each other,
wherein the annular member is formed by disposing a plurality of basic units in a circumferential direction of the cylindrical shape and connecting the basic units adjacent to each other by a second bent portion,
the basic unit including:
a wavy unit which has a plurality of first linear portions extending from a proximal end side to a distal end side in the long-axis direction and disposed continuously in the circumferential direction and a first bent portion connecting end portions on the proximal end side or the distal end side of two of the first linear portions adjacent in the circumferential direction;
a second linear portion extending from the proximal end side to the distal end side in the long-axis direction and disposed at a position adjacent to the first linear portion in the circumferential direction; and
the second bent portion connecting end portions on the proximal end side or the distal end side of the first linear portion and the second linear portion adjacent to each other in the circumferential direction,
a second distance, which is a separation distance in the circumferential direction between an extreme end portion in the long-axis direction of the second bent portion connected to an end portion on one end side of the second linear portion and an extreme end portion in the long-axis direction of the second bent portion connected to an end portion on another end side of the second linear portion, is longer than a first distance which is a separation distance in the circumferential direction between an extreme end portion in the long-axis direction of the first bent portion connected to an end portion on one end side of any of the first linear portions and an extreme end portion in the long-axis direction of the first bent portion or the second bent portion connected to an end portion on another end side of the first linear portion, and
a number of the first linear portions disposed in the wavy unit is four or more.

2. The stent according to claim 1, wherein the number of the first linear portions is an even number and a number of the second linear portions is one in the basic unit.

3. The stent according to claim 2, wherein the number of the first linear portions is four.

4. The stent according to any one of claims 1 to 3, wherein a line width of the first bent portion is smaller than a line width of the first linear portion connected to the first bent portion.

5. The stent according to claim 4, wherein a line width of the second bent portion is smaller than line widths of the first linear portion and the second linear portion connected to the second bent portion.

6. The stent according to any one of claims 1 to 5, wherein a second radius, which is a radius of curvature on an inner curved side of the second bent portion, is larger than a first radius which is a radius of curvature on an inner curved side of the first bent portion.

7. The stent according to claim 6, wherein a radius of curvature on an inner curved side of the first bent portion to which the link portion is connected is larger than a radius of curvature on an inner curved side of the first bent portion to which the link portion is not connected.

8. The stent according to any one of claims 1 to 7, wherein
the first linear portion has an axis-parallel linear portion parallel to the long-axis direction,
the annular members are disposed with aligned phases in the circumferential direction,
the link portion connects the first bent portion connected to a distal end side of the axially parallel linear portion of the annular member disposed on the proximal end side and the first bent portion connected to a proximal end side of the axially parallel linear portion of the annular member disposed on the distal end side and adjacent to the annular member disposed on the proximal end side, and
a portion from a proximal end side of the axially parallel linear portion of the annular member on the proximal end side to a distal end side of the axially parallel linear portion of the annular member on the distal end side via the link portion is parallel to the long-axis direction before and after expansion.

9. The stent according to any one of claims 1 to 8, wherein
a pair of the link portions is disposed in each of gaps between the adjacent annular members to face each other in a radial direction of the annular member, and
a phase of the link portion disposed in one gap and a phase of the link portion disposed in another gap adjacent to the one gap in the long-axis direction are shifted by 90° in the circumferential direction.

10. A stent delivery system comprising:
the stent according to any one of claims 1 to 9; and
a balloon catheter having an inflatable and deflatable balloon,
wherein the stent is retained in close contact with the balloon, which has been deflated, in a contracted state, and
the balloon protrudes outward from a radial position of an inner surface of the stent only at a position between the first linear portion and the second linear portion.
